# EUROPÄISCHE PATENTANMELDUNG

(11) **EP 0 700 691 A1**
(43) Veröffentlichungstag der Anmeldung: **13.03.1996**
(21) Anmeldenummer: 95113651.4
(22) Anmeldetag: 31.08.1995
(51) Int. Cl.: A61M 25/01, A61M 39/00, A61M 5/158

(54) **Sicherheitseinrichtung für Gefässzugänge**

(30) Priorität: 12.09.1994 DE 4432348
(71) Anmelder: Borger, Ludwig, D-45711 Datteln (DE)
(72) Erfinder: Borger, Ludwig, D-45711 Datteln (DE)
(74) Vertreter: Eichelbaum, Lambert, Dipl.-Ing.

(57) **Zusammenfassung**

Gefäßzugangssicherung von blut-, wundsekret-, ernährungs- oder infusionsführenden Gefäßzugängen. Hierbei ist am Schlauch einer Kanüle oder Katheters ein Magnet (1) angebracht und unabhängig davon ein Reed-Relais (2) so fixiert, daß beim Herausrutschen des Gefäßzuganges aus dem Gefäß sich der Magnet (1) vom Reed-Relais (2) entfernt und somit ein Überwachungsgerät (3) alarmiert oder ein Behandlungsgerät (3) zum Stillstand gebracht wird.

## Beschreibung

Die Erfindung betrifft eine Gefäßzugangssicherung von blut-, wundsekret-, ernährungs- oder infusionsführenden Gefäßzugängen.

Bisher gibt es keine wirksame Sicherung von Gefäßzugängen. Zur Zeit behilft man sich lediglich durch Verkleben der Zugänge mit Pflastern oder, bei den zentralen Venenkathetern, mit dem Annähen.

Außerdem liegt hierbei kein unmittelbares technisches Überwachungssystem vor. Dadurch kann ein Herausrutschen von Kanülen und Kathetern vorkommen, was in der Regel nicht sofort erkannt wird und zu folgenden, teils erheblichen Gefährdungen des Patienten führen kann:
1. Luftembolie bei zentralen Venenkathetern.
2. Blutverluste bei Transfusionen oder bei Blutrückgabe.
3. Kontamination des Gefäßzuganges.

Nachfolgend einige Beispiele für die 3 erwähnten Hauptgruppen von Gefährdungen:
Zu 1.: Beim Zusammentreffen von einem negativen zentralen Venendruck und dem teilweisen Herausrutschen eines zentralen Venenkatheters kommt es zu einer Luftembolie, bei der Luft in den Kreislauf des Patienten eindringt, die im Herzen oder in der Lunge zu einer Verlegung von Kapillarbereichen führt und tödlich enden kann.
Zu 2.: Herkömmliche Dialysegeräte messen bei der Rückgabe des Blutes den Widerstand zwischen Gerät und Patient. Dabei wird das Blut mit einer Geschwindigkeit von 200-400 ml/min durch eine Kanüle in den Patienten gefördert. Allein der Widerstand der Kanüle liegt dabei größtenteils im Drucküberwachungsbereich des Dialysegerätes. Beim Herausrutschen der Kanüle fließt dann Blut aus dem arteriellen Gefäßzugang und dem Dialysegerät außerhalb des Patienten. Das Dialysegerät reagiert erst aufgrund sekundärer Einflüsse.
Zu 3.: Blut-, wundsekret- oder infusionsführende Gefäßzugänge können herausrutschen. Hygienisch können an diesen ungeschützten Gefäßzugängen mikrobielle Verunreinigungen stattfinden.

Der Erfindung liegt die Aufgabe zugrunde, ein einfach zu bedienendes Überwachungssystem zu schaffen. Die Lösung dieser Aufgabe erfolgt durch eine technische Vorrichtung, die mit einem eigenen oder bereits beim Patienten angeschlossenen Überwachungsgerät gekoppelt ist.

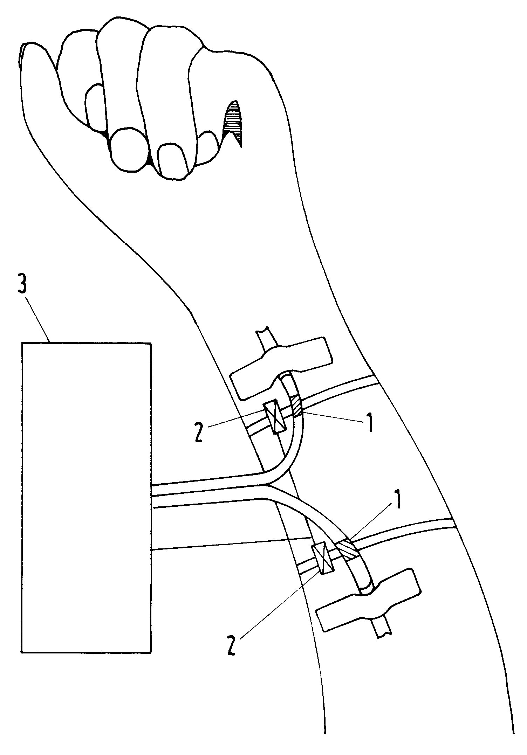

Ein Ausführungsbeispiel der Erfindung ist in der Figur 1 dargestellt.

Durch die neuartige Sicherungsvorrichtung werden alle erwähnten Sicherheitsmängel beseitigt, weil: Die Gefäßzugangssicherung am Schlauch der Kanüle oder des Katheters in unmittelbarer Nähe des Gefäßzuganges oder an einer anderen Stelle einen befestigten Magneten 1 aufweist und ein Reed-Relais 2, ein Schutzkontakt-Relais mit elektromagnetischer Erregung, unabhängig so fixiert wird, daß beim Entfernen des Gefäßzuganges aus dem Gefäß sich der Abstand des Reed-Relais 2 vom Magneten 1 vergrößert und ein angeschlossenes Überwachungsgerät 3 alarmiert oder entsprechende Behandlungsgeräte 3 reagieren, z.B. durch Stillstand der Blutpumpe bei Dialysegeräten.

### Bezugszeichenliste:

- Magnet: 1
- Reed-Relais: 2
- Behandlungsgerät: 3

## Patentansprüche

1. Gefäßzugangssicherung von blut-, wundsekret-, ernährungs- oder infusionsführenden Gefäßzugängen, **dadurch gekennzeichnet,** daß ein Magnet (1) am Schlauch der Kanüle oder des Katheters am Produkt direkt oder separat und individuell, falls sinnvoll und erfolgreich, in unmittelbarer Nähe des Gefäßzuganges oder an einer anderen Stelle befestigt ist, und ein Reed-Relais (2) unabhängig vom Magneten (1) fixiert wird, daß beim Entfernen des Gefäßzuganges aus dem Gefäß sich der Abstand des Reed-Relais (2) vom Magneten (1) vergrößert und ein angeschlossenes Überwachungsgerät (3) alarmiert oder entsprechende Behandlungsgeräte (3) reagieren, indem diese Geräte (3) Alarm geben und die blut-, wundsekret-, ernährungs- oder infusionsführende Tätigkeit unterbrechen.
